# EUROPEAN PATENT APPLICATION

(11) **EP 2 742 934 A1**
(43) Date of publication of application: **18.06.2014**
(21) Application number: 14158985.3
(22) Date of filing: 01.10.2009
(51) Int. Cl.: A61K 9/70, A61K 31/381

(54) **Amorphous rotigotine transdermal system**

(30) Priority: 06.10.2008 US 195319 P
(62) Divisional of application: 09749216.9
(71) Applicant: MYLAN TECHNOLOGIES, INC., St. Albans, VT 05478 (US)
(72) Inventor: Tang, Jiansheng, South Burlington, VT 05403 (US); Deverich, Joseph M., Essex Junction, VT 05452 (US); Miller, Kenneth J. II, St. Albans, VT 05478 (US); Beste, Russell D., South Burlington, VT 05403 (US)
(74) Representative: Hamer, Christopher K.

(57) **Abstract**

The present invention refers to a transdermal device comprising a backing layer, an adhesive matrix layer comprising a supersaturated concentration of rotigotine substantially in amorphous form within the adhesive matrix, and a release liner. The present invention also refers to a method of preparing an adhesive matrix containing a supersaturated amount of rotigotine substantially in amorphous form. Further, the present invention refers to a method of stabilizing and a method of re-establishing the meta-stable amorphous-drug transdermal system during its manufacturing, storing, shipping and handling process.

## Description

### CROSS-REFERENCE TO RELATED APPLICATIONS

This application claims the benefit of the filing date of United States Provisional Patent Application No. 61/195,319 filed October 6, 2008, the disclosure of which is hereby incorporated herein by reference.

### BACKGROUND OF THE INVENTION

The present invention relates to transdermal drug delivery systems.

The delivery of drugs through the skin provides many advantages. Primarily, it is a comfortable, convenient and non-invasive way of administering drugs. Moreover, such a means of delivery provides for uninterrupted therapy and a higher degree of control over drug concentrations in the blood.

United States Patent No. 5,164,190 discloses transdermal administration of hydrophobic drugs via a diffusion mechanism in which the drug is dissolved in a carrier at concentrations between 20% and 80% of saturation concentration. This patent, however, fails to suggest an amorphous transdermal drug delivery system in which the drug is supersaturated and in which the supersaturated portion of the drug is present in an amorphous drug-in-adhesive matrix.

United States Patent No. 4,409,206 discloses a preparation in the form of a polyacrylate film with an amorphous active pharmaceutical ingredient embedded therein. This patent does not, however, disclose a transdermal delivery device or a system containing a supersaturated concentration of an amorphous drug within an adhesive matrix.

United States Publication No. 2005/0064022 describes a device comprising amorphous terazosin. More specifically, the publication discloses a transdermal therapeutic system for the administration of amorphous terazosin to the skin comprising a backing layer, a pressure-sensitive adhesive reservoir layer and/or a matrix layer, and optionally a removable protective layer.

United States Publication No. 2005/0175678 A1 is directed to a polymer matrix suitable for the transdermal administration of rotigotine and a method of preparing the same. The polymer matrix contains a supersaturated amount of a rotigotine base such that the part of the rotigotine that is not dissolved in the matrix polymer is dispersed in the matrix as amorphous particles. The publication further discloses that the matrix may be a component of a system for transdermal administration of rotigotine, wherein the system can have components such as a protective layer, a backing layer, further polymer layers, and/or a membrane which controls release of the rotigotine.

United States Patent No. 6,902,741 is directed to a transdermal system which includes a sex hormone-containing adhesive matrix, containing inclusions of sex hormone in a hydrophilic non-crosslinked polymer. The active substance contained in the inclusions is preferably amorphous to an extent of more than 50% by weight of the active substance. The active substance-containing laminate is characterized in that the active substance inclusions are contained in the adhesive matrix in dissolved or dispersed form.

Various methods of manufacturing transdermal systems in which the drug is supersaturated are known. United States Patent Nos. 4,409,206, 4,490,322, 4,797,284, 4,880,633, 5,352,457 5,869,089, 5,906,830, 6,153,216, 6,156,335, and 6,623,763 describe methods of manufacturing transdermal systems. United States Patent No. 4,490,332 discloses a method of manufacturing a polyacrylate film for long term transdermal administration by forming a solution of a pharmaceutical and a freeze-dried latex polyacrylate copolymer in a solvent. United States Patent No. 5,906,830 discloses a method of manufacturing a supersaturated transdermal system comprising heating a mixture of undissolved drug and reservoir matrix material to a predetermined temperature, followed by cooling. These references, however, fail to disclose a method of making a stable transdermal device containing an active agent in amorphous form.

Finally, one problem encountered with drug delivery devices comprising supersaturated solutions is insufficient storage stability due to crystallization processes. Such crystallization processes result in a reduction in the amount of dissolved drug, and an increase in the amount of drug present in the crystalline state, thus reducing the efficacy of such a supersaturated device. To prevent crystallization processes in transdermal delivery devices and to be able to administer the therapeutically desired dose continuously, crystallization inhibitors are usually added to any delivery device. United States Patent Nos. 6,465,005, 5,676,968, 6,440,454, and 6,537,576 describe methods utilizing such crystallization inhibitors. However, the addition of non-adhesive crystallization inhibitors alters the adhesion properties of the adhesive by reducing its adhesiveness or by making the system softer. As such, the prior art fails to suggest a method of stabilizing an amorphous drug-in-adhesive matrix delivery device. Moreover, the prior art fails to suggest a method of reestablishing an amorphous drug-in-adhesive delivery device.

### SUMMARY OF THE INVENTION

In accordance with the present invention, a transdermal delivery device has been discovered comprising a backing layer, a release liner, and an adhesive matrix layer between the backing layer and the release liner comprising a supersaturated concentration of rotigotine substantially in amorphous form within the adhesive matrix, wherein the backing layer is the same size as or larger than the adhesive matrix layer.

In accordance with another embodiment of the present invention, the rotigotine is present in an amount of about 0.1% to about 50% by weight of the adhesive matrix. In accordance with another embodiment of the present invention, the concentration of the rotigotine is from about 0.1% to about 10000% above the solubility of the active agent in the adhesive matrix.

In accordance with another embodiment of the present invention, the backing layer and the release liner are substantially non-crystallization inducing and free of crystallization nuclei or crystallization seeding particles. In accordance with another embodiment of the present invention the backing layer is selected from the group consisting of polyester films, polyethylene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films. In accordance with another embodiment of the present invention, the backing layer is polyester.

In accordance with another embodiment of the present invention, the backing layer is at least about 0.01mm larger than the adhesive matrix layer. In accordance with another embodiment of the present invention, the backing layer is about 0.01mm to about 10mm larger than the adhesive matrix layer. In accordance with another embodiment of the present invention, the backing layer is about 0.05mm to about 5mm larger than the adhesive matrix layer. In accordance with another embodiment of the present invention, the backing layer is about 0.1mm to about 3mm larger than the adhesive matrix layer.

In accordance with another embodiment of the present invention, the release liner is larger than the adhesive matrix layer. In addition to the protective release liner, shipping pouches may be used as a further means to protect the device.

In accordance with another embodiment of the present invention, the adhesive matrix layer comprises an adhesive material selected from the group consisting of polyisobutylene, polysiloxane, acrylic adhesives, natural and synthetic rubber adhesives, and mixtures thereof. In accordance with another embodiment of the present invention the adhesive material is present in an amount of from about 50% to about 99% by weight of the adhesive matrix layer.

In accordance with another embodiment of the present invention, the adhesive matrix layer further comprises one or more tackifiers. In accordance with another embodiment of the present invention, the adhesive matrix layer further comprises one or more cohesive enhancers. In accordance with another embodiment of the present invention, the adhesive matrix layer further comprises one or more flux enhancers.

In accordance with another embodiment of the present invention, the device further comprises a drug release regulating membrane layer and/or a reservoir layer. In accordance with another embodiment of the present invention, at least one of the drug release regulating membrane layer and/or the reservoir layer contains rotigotine. Of course, either of these layers may be the same size or larger than the backing layer, reservoir layer, or adhesive matrix layer(s).

In accordance with another embodiment of the present invention, the device further comprises an overlay film in communication with the backing layer. In accordance with another embodiment of the present invention, the overlay film is larger than the backing layer. In accordance with another embodiment of the present invention, the overlay film is about 0.01mm to about 20mm larger than the backing layer.

In accordance with the present invention, a transdermal delivery device has been discovered comprising an overlay film, a backing layer adjacent to the overlay film, an adhesive matrix layer adjacent to the backing layer comprising a supersaturated concentration of rotigotine substantially in amorphous form within the adhesive matrix, and a release liner adjacent to the adhesive matrix layer.

In accordance with another embodiment of the present invention, the overlay film is larger than the backing layer. In accordance with another embodiment of the present invention, the overlay film is at least 0.01mm larger than the backing layer. In accordance with another embodiment of the present invention, the overlay film is between about 0.01mm to about 20mm larger than the backing layer. In accordance with another embodiment of the present invention, the overlay film covers at least one edge of the backing layer.

In accordance with another embodiment of the present invention, the overlay film is selected from the group consisting polyester films, polyurethane films, polyester films with a silicone coating, polyurethane films with a silicone coating, polyester films with a fluorosilicone coating, polyurethane films with a fluorosilicone coating, silicon coated polyester films, silicon coated polyurethane films, polyester films with a fluoropolymer coating, and polyurethane films with a fluoropolymer coating.

In accordance with another embodiment of the present invention, the backing layer and the release liner are substantially non-crystallization inducing and free of crystallization nuclei or crystallization seeding particles.

In accordance with another embodiment of the present invention, the backing layer is selected from the group consisting of polyester films, polyethylene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films.

In accordance with another embodiment of the present invention, the rotigotine is present in an amount of about 0.1% to about 50% by weight of the adhesive matrix.

In accordance with another embodiment of the present invention, the at least one of the backing layer or release liner is larger than the adhesive matrix layer. In accordance with another embodiment of the present invention, the backing layer is the same size as the adhesive matrix layer. In accordance with another embodiment of the present invention, the backing layer is larger than the adhesive matrix layer. In accordance with another embodiment of the present invention, the release liner is larger than the adhesive matrix layer.

In accordance with another embodiment of the present invention, the adhesive matrix layer comprises an adhesive material selected from the group consisting of polyisobutylene, polysiloxane, acrylic adhesives, natural and synthetic rubber adhesives, and mixtures thereof. In accordance with another embodiment of the present invention, the adhesive material is present in an amount of from about 50% to about 99% by weight of the adhesive matrix layer.

In accordance with another embodiment of the present invention, the device further comprises a drug release regulating membrane layer and/or a reservoir layer.

In accordance with the present invention, a transdermal delivery device has been discovered comprising a polyester backing layer, a substantially non-crystallization inducing release liner, and an adhesive matrix layer between the backing layer and the release liner comprising a supersaturated concentration of rotigotine substantially in amorphous form within the adhesive matrix.

In accordance with another embodiment of the present invention, at least one of the polyester backing layer or the release liner is larger than the adhesive matrix layer. In some embodiments, both are larger.

In accordance with another embodiment of the present invention, the device further comprises an overlay film adjacent to the polyester backing layer and opposite the adhesive matrix layer. In accordance with another embodiment of the present invention, the overlay film is larger than the polyester backing layer.

In accordance with the present invention, a transdermal delivery has been discovered comprising an overlay film, a substantially non-crystallization inducing backing layer adjacent to the overlay film, an adhesive matrix layer adjacent to the backing layer comprising a supersaturated concentration of rotigotine substantially in amorphous form within the adhesive matrix, and a substantially non-crystallization inducing release liner adjacent to the adhesive matrix layer, wherein at least one of the overlay film, backing layer, or release liner is larger than the adhesive matrix layer.

In accordance with another embodiment of the present invention, the backing layer is larger than the adhesive matrix layer. In accordance with another embodiment of the present invention, the overlay film is larger than the adhesive matrix layer. In accordance with another embodiment of the present invention, the release liner is larger than the adhesive matrix layer.

In accordance with another embodiment of the present invention, at least one of the overlay film, backing layer, or release liner is larger than the adhesive matrix layer in at least one dimension.

In accordance with another embodiment of the present invention, the backing layer is selected from the group consisting of polyester films, polyethylene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films.

In accordance with the present invention, a transdermal delivery device has been discovered comprising a substantially non-crystallization inducing backing layer and an adhesive matrix layer adjacent to the backing layer comprising a supersaturated concentration of rotigotine substantially in amorphous form within the adhesive matrix layer, wherein the backing layer is larger than the adhesive matrix layer.

In accordance with another embodiment of the present invention, the device further comprises a substantially non-crystallization inducing release liner adjacent to the adhesive matrix layer.

In accordance with another embodiment of the present invention, the device is stored, transported or protected in protective packaging prior to use. In accordance with another embodiment of the present invention, the protective packaging is a pouch comprised of paper, polymer film, metal foil or combinations thereof.

In accordance with another embodiment of the present invention, the device further comprises an overlay film adjacent to the backing layer and opposite the adhesive matrix layer.

In accordance with another embodiment of the present invention, the overlay film is larger than at least one of the backing layer or the adhesive matrix layer.

In accordance with the present invention, a transdermal delivery device has been discovered comprising a substantially non-crystallization inducing backing layer, a substantially non-crystallization inducing release liner, and an adhesive matrix layer between the backing layer and the release liner comprising a supersaturated concentration of rotigotine substantially in amorphous form within said adhesive matrix, at least one of the backing layer or release liner is larger than the adhesive matrix layer, and wherein the transdermal delivery device is cured at a temperature above the melting point of the rotigotine.

In accordance with another embodiment of the present invention, the curing is performed at a temperature about 20°C above the melting point of the rotigotine. In accordance with another embodiment of the present invention, the curing is performed for a duration ranging from about 1 second to about 10 minutes. In accordance with another embodiment of the present invention, the duration ranges from about 3 seconds to about 5 minutes. In accordance with another embodiment of the present invention, the device further comprises an overlay film adjacent to the backing layer and opposite the adhesive matrix layer.

In accordance with another embodiment of the present invention, the overlay film is larger than the adhesive matrix layer.

In accordance with the present invention is a method of preparing an adhesive matrix comprising rotigotine that is supersaturated and present in amorphous form comprising the steps of:
a) dissolving rotigotine and an adhesive polymer in a solvent in an amount so as to provide rotigotine at a subsaturated concentration in an adhesive matrix solution,
b) casting the subsaturated rotigotine in the adhesive matrix solution to one of a release liner and a backing layer,
c) removing the solvent at a temperature which is at, below, or above the melting point of the active agent to form a dry adhesive matrix in which rotigotine is in a supersaturated concentration, and d) laminating the other of the release liner and the backing film to the supersaturated rotigotine in the dry adhesive matrix, so that the supersaturated rotigotine in the dry adhesive matrix is between the release liner and the backing layer.

In accordance with the present invention, a method of preparing an adhesive matrix containing at least one active agent that is supersaturated and present in amorphous form has been discovered comprising the steps of: a) admixing the active agent with an adhesive matrix at a supersaturated concentration, b) heating the supersaturated concentration of the active agent in the adhesive matrix to a temperature which allows the active agent to be completely dissolved and uniformly dispersed in the adhesive matrix to create a hot melt, c) casting the hot melt to one of a release liner and a backing layer, at a predetermined temperature, and d) laminating the other of the release liner and the backing layer to the hot melt, so that the hot melt is between the release liner and the backing layer. Here, the active agent is rotigotine.

In accordance with another embodiment of the present invention, is a method of reestablishing the favored internal adhesive matrix environment of a transdermal drug delivery device having a backing layer, an adhesive matrix layer having a supersaturated concentration of rotigotine substantially in the amorphous form within the adhesive matrix layer, and a release liner, comprising curing the transdermal delivery device. In accordance with another embodiment of the present invention, the heat curing comprises heating the transdermal delivery device to a temperature at which rotigotine completely dissolves or to a temperature about 20°C above the melting point of rotigotine. In accordance with another embodiment of the present invention, the curing comprises subjecting the device to oven infrared beams. In accordance with another embodiment of the present invention, the curing is performed for a duration ranging from about 1 second to about 10 minutes, preferably ranging from about 3 seconds to about 5 minutes, most preferably ranging from about 5 seconds to about 60 seconds.

In accordance with yet another embodiment of the present invention, is a method of storing and protecting a transdermal delivery device having a backing layer, an adhesive matrix layer comprising a supersaturated concentration of rotigotine substantially in amorphous form within the adhesive matrix, and a release liner wherein the method comprises packaging the transdermal delivery device in a pouch. The pouch may be the same size or larger than the release liner. The pouch may be comprised of paper, polymer film(s), metal foil(s), or any combination thereof.

Applicants have found, unexpectedly, that an amorphous-drug-in-adhesive provides a higher skin flux relative to transdermal delivery devices containing crystalline forms of rotigotine (alone or in combination with other active agents) in a subsaturated solution. Further, Applicants have discovered a method of forming transdermal delivery devices incorporating the amorphous form of rotigotine which is typically very difficult to stabilize.

### DETAILED DESCRIPTION

Generally, the present invention is directed to a transdermal delivery device (or "patch") comprising a backing layer, an adhesive matrix layer comprising a supersaturated concentration of at least one active agent substantially in amorphous form within an adhesive matrix, and a release liner.

As used herein, "transdermal" means delivery of a drug by passage into and through the skin or mucosal tissue. Hence the terms "transdermal" and "transmucosal" are used interchangeably unless specifically stated otherwise. Likewise the terms "skin," "derma," "epidermis," "mucosa," and the like shall also be used interchangeably unless specifically stated otherwise.

The backing layer is a flexible substrate which provides a barrier to active drug migration away from the intended direction of drug delivery. Any well-known backing layer which satisfies this purpose can be used in the present invention.

In some embodiments, the backing layer is composed of materials that are substantially non-crystallization promoting and free of crystallization nuclei. Such backing layers aid in the preservation of the amorphous drug-in-adhesive matrix by preventing crystal formation.

Examples of materials from which the backing layer may be composed include polyethylene terephthalate, various nylons, polypropylenes, polyesters, polyester/ethylene-vinyl acetates, metalized polyester films, polyvinylidene chloride, metal films such as aluminum foils, polyvinylidene fluoride films, or mixtures or copolymers thereof.

Other materials for the backing layers include ethylene vinyl acetate films laminated to a polyester, ethylene vinyl acetate films laminated to a metalized polyester, Mediflex® 1200 available from Mylan Technologies, Inc., Mediflex® 1501 from Mylan Technologies Inc., Mediflex® 1201 available from Mylan Technologies, Inc., Mediflex® 1502 available from Mylan Technologies, Inc., Dupont polyester type S available from Dupont, Dow BLF® 2050 available from The Dow Chemical Company, 3M™ Scotchpak® 1109 available from 3M, 3M™ Scotchpak® 9723 available from 3M, 3M™ Scotchpak® 9733 available from 3M, 3M™ Scotchpak® 9735 available from 3M and 3M™ Scotchpak® 9730 available from 3M.

Silicone coated polyethylene backings, such as Mediflex® 1000 coated with a silicone layer, 3M™ Cotran® 9722 coated with a silicone layer, and 3M™ Cotran™ 9720 coated with a silicone layer, preserve the amorphous form of the drug in the adhesive matrix. Similarly, silicone coated polyester backings, such as Mediflex® 1200 coated with a silicone layer, also preserves the amorphous form of drug in adhesive.

In preferred embodiments, the backing layer is comprised of polyester, a polyester derivative, a polyester based copolymer, or a polyester blend, collectively referred to as "polyester."

In some embodiments, the backing layer may be the same size as the adhesive matrix layer and/or may be the same size as the release liner.

In other embodiments, the backing layer may be oversized as compared with the adhesive layer, i.e. the backing layer may be larger than the adhesive layer.

In yet other embodiments, the backing layer may range from about 0.01mm to at least 10mm larger than the adhesive matrix layer. In further embodiments, the backing layer may range from about 0.05mm to about 5mm larger than the adhesive matrix layer. In yet further embodiments, the backing layer may range from about 0.1mm to about 3mm larger than the adhesive matrix layer.

Without wishing to be bound by any particular theory, it is believed that the use of an oversized backing layer helps prevent the adhesive matrix from becoming distorted or relaxing during the handling and/or shipping processes. Indeed, use of an oversized backing layer may help prevent crystal growth, especially when the devices are stored for long periods of time or when they are exposed to temperature fluctuations or other environmental stresses.

Adjacent to the backing layer is an adhesive matrix layer comprising a supersaturated concentration of at least one active agent dissolved and/or dispersed in an adhesive material.

The "adhesive material" or "adhesive matrix" (used interchangeable) may be any biocompatible polymer or polymeric material known in the art. The adhesive matrix material may be selected from silicones, natural and synthetic rubbers, polyisobutylene ("PIB"), neoprenes, polybutadienes, polyisoprenes, polysiloxanes, acrylic adhesives including cross-linked and uncross-linked acrylic copolymers, vinyl acetate adhesives, polyacrylates, ethylenevinylacetate copolymers, styrene-isoprene copolymers, polyurethanes, plasticized weight polyether block amide copolymers, plasticized styrene-rubber block copolymers, and mixtures thereof.

The adhesive matrix material may also be selected from acrylic adhesives and polyacrylate adhesives sold under the trademark Duro-Tak 80-1194, 80-1196,80-1197,2287,2516 2852, 387-2051, 387-2052, 387-2054, 387-2287, 387-2353, 387-2510, 387-2516, 387-2620, 387-2825, 387-2070, 87-2074, 87-2097, 87-2100, 87-2154, 87-2194, 87-2196, 87-2852 and 87-2979 by National Starch and Chemical Corporation, Bridgewater, N.J., USA. Other suitable acrylic adhesives include those sold under the trademark Gelva--Multipolymer Solution GMS 737, 788, 263, 1151, 1159, 1430, 1753, 2450, 2465, 2480, 2495, 2497 and 2539 by Monsanto, St Louis, Mo. USA.

Pressure sensitive silicone containing adhesives are available from Dow Corning under the trademark BIO-PSA® 7-4101, 7-4201, 7-4301, 7-4102, 7-4202, 7-4302, 7-4103, 7-4203, and 7-4303 and may be utilized as an adhesive matrix material.

In some embodiments, the adhesive matrix material is generally present in the adhesive matrix layer in an amount ranging from about 50% to about 99% by weight of the adhesive matrix layer. In other embodiments, the adhesive matrix material is present in the adhesive matrix layer in an amount ranging from about 60% to about 90% by weight of the adhesive matrix layer.

The active agent is dissolved or dispersed within the adhesive matrix and present substantially in amorphous form. As used herein, the terms "active agent," "active pharmaceutical ingredient," "API," or "drug" (used interchangeably) are used to describe the principal active pharmaceutical ingredient of the transdermal delivery device, which is a biologically active compound or mixture of compounds that has a therapeutic, prophylactic and/or physiological effect on the wearer of the device.

As used herein, the term "substantially" means to meet the criteria in such measure that one skilled in the art would understand that the benefit to be achieved, or the condition or property value desired, is met. In some embodiments, at least 40% of the active agent is present in amorphous form. In other embodiments, at least 50% of the active agent is present in amorphous form. In yet other embodiments, at least 60% of the active agent is present in amorphous form. In further embodiments, at least 75% of the active agent is present in amorphous form.

The active agent may be any active pharmaceutical ingredient capable of being provided in an amorphous form within a transdermal delivery device. The active agent may be a mixture of APIs, provided that each of the APIs is present substantially in amorphous form.

Non-limiting examples of active agents include anti-inflammatory substances, opioid receptor antagonists, anticholinergics, coronary dilators, cerebral dilators, peripheral vasodilators, alpha-adrenergic blockers, anti-infectives, psychotropics, anti-manics, stimulants, anti-histamines, decongestants, gastro-intestinal sedatives, anti-anginal drugs, vasodilators, anti-arrhythmics, anti-hypertensive drugs, vasoconstrictors, migraine treatments, anti-coagulants and anti-thrombotic drugs, analgesics, anti-pyretics, hypnotics, sedatives, anti-emetics, anti-nauseants, anti-convulsants, neuromuscular drugs, hyper-and hypoglycemic agents, thyroid and anti-thyroid preparations, diuretics, anti-spasmodics, anti-emetic, uterine relaxants, anti-obesity drugs, anabolic drugs, erythropoietic drugs, anti-asthmatics, bronchodilators, expectorants, mucolytics, anti-uricemic drugs, narcotics, anti-depressants, agents for treating alcohol abuse or dependence and the like.

In some embodiments of the present invention, the active agent is rotigotine. As used herein, the term "rotigotine" is used to designate rotigotine, the salts, solvates, and hydrates of rotigotine, and the related compounds, derivatives, or analogs thereof. In a preferred embodiment, the active agent is rotigotine in the form of a free base. In some embodiments, the rotigotine is mixed with another API, provided that the other API is substantially in amorphous form.

In some embodiments, the active agent is present in an amount ranging from about 0.1% to about 50% by weight of the adhesive matrix layer. In other embodiments, the active agent is present in an amount ranging from about 1% to about 20% by weight of the adhesive matrix layer.

The active agent is present in a supersaturated concentration within the adhesive matrix. In some embodiments, the active agent concentration ranges from about 0.1% to 10000% above the solubility of the active agent in the adhesive matrix. In other embodiments, the active agent concentration ranges from about 5% to about 5000% above the solubility of the active agent in the adhesive matrix. In yet other embodiments, the concentration of active agent ranges from about 10% to about 1000% above the solubility of the active agent in the adhesive matrix.

In some embodiments, the amount of active agent present in amorphous form within the device is generally in an amount ranging from about 1% to about 100% by weight of the total amount of active agent, preferably ranging from about 20% to about 80% by weight of the total amount of active agent, and most preferably ranging from about 40% to about 60% by weight of the total amount of active agent.

The adhesive matrix layer may contain one or more additives selected from tackifiers, cohesive enhancers, permeation enhancers, crystal growth inhibitors, plasticizers, antioxidants, flux enhancers, penetration enhancers, and/or other pharmaceutically acceptable additives or excipients. The additives are generally present in the composition in an amount ranging from about 1% to about 50% by weight of the adhesive matrix layer, and preferably ranging from about 2% to about 25% by weight of the adhesive matrix layer.

In some embodiments, the adhesive matrix layer contains one or more tackifiers. As used herein, the term "tackifier" refers to materials other than PIB that are added to adhesives to increase their tack or stickiness. If tackifiers are included, they are generally present in an amount ranging from about 1% to about 50% by weight of the adhesive matrix layer, preferably from about 5% to about 40% by weight of the adhesive matrix layer. Tackifiers are generally comprised of materials such as naturally occurring resinous, rosinous materials, or truly synthetic polymer materials. Examples of tackifiers include hydrogenated or partially hydrogenated glycerol esters of rosin, polyterpenes, polybutenes, or polysiloxanes.

In some embodiments, the adhesive matrix layer contains one or more cohesive enhancers. The addition of a cohesive enhancer into the adhesive matrix increases the adhesive matrix's storage modulus. Cohesive enhancers are generally present in an amount ranging from about 0.1% to about 25% by weight of the adhesive matrix layer, preferably from about 1% to about 15% by weight of the adhesive matrix layer. Examples of cohesive enhancers include colloidal silicone dioxide, zinc oxide, clays, bentonite, polyvinylpyrrolidone ("PVP"), polyvinylpyrrolidone-co-vinylacetate, Eudragit® copolymers (available from Evonik Industries AG, Rellinghauser Strabe 1-11, 45128 Essen, Germany), ethyl cellulose or crosspovidone.

In some embodiments, the adhesive matrix layer contains one or more flux enhancers as part of the drug formulation. As used herein, the term "flux enhancer" is used to describe a compound which aids in increasing the permeability of a drug through the skin to the blood stream. If flux enhancers are included, they are generally present in an amount ranging from about 0.1% to about 40% by weight of the adhesive matrix layer, preferably from about 1% to about 20% by weight of the adhesive matrix layer.

Suitable flux enhancers include dimethylsulfoxide (DMSO), dimethyl formamide (DMF), N,N-dimethylacetamide (DMA), decylmethylsulfoxide, polyethylene glycol monolaurate (PEGML), propylene glycol (PG), propylene glycol monolaurate (PGML), butylene glycol, dipropylene glycol, diethylene glycol, propyl palmitate, isopropyl palmitate, propyl myristate, glycerol monoesters, glycerol monolaurate (GML), propylene glycol monoester, polyethylene glycol monoester, methyl laurate (ML), lauryl lactate (LL), isopropyl myristate (IPM), terpenes such as menthone, C₂-C₆ diols, particularly 1,2-butanediol, lecithin, the 1-substituted azacycloheptan-2-ones, 1-n-dodecylcyclazacycloheptan-2-one, C₂ to C₁₈ alcohols, triacetin, and the like. Vegetable oil permeation enhancers, as described in United States Patent No. 5,229,130, may also be used. Such oils include safflower oil, cotton seed oil and corn oil.

Adjacent to the adhesive matrix layer is an optional release liner. Release liners well known in the art can be used in the present invention. Examples of materials from which the release liner may be composed include polyethylene terephthalate/silicone (i.e. polydimethyl siloxane) ("PET/SI"), polyethylene terephthalate/aluminized polyester coated with silicone (i.e. polydimethyl siloxane) ("PET/MET/SI"), polyester or polyurethane liners with a silicone coating, polyester or polyurethane liners with a fluorosilicone coating, or polyester or polyurethane liners with a silicon coating.

Preferably, the release liner is composed of materials that are substantially non-crystallization promoting and free of crystallization nuclei. Such release liners aid in the preservation of the amorphous drug-in-adhesive matrix. Specific release liners include Medirelease® 2249, Medirelease® 2226, Medirelease® 2500, 3M™ Scotchpak® 1020, 3M™ Scotchpack® 1022, 3M™ Scotchpak® 9741, 3M™ Scotchpak® 9742, 3M™ Scotchpak® 9744, CPFilms Inc. Clearsil® UV5A and CPFilms Inc., Clearsil® UV510, CPFilms Inc. Sil® UV5A and CPFilms Inc. Sil® UV510.

In some embodiments, the release liner may be the same size as the adhesive matrix layer and/or may be the same size as the backing layer. In other embodiments, the release liner may be larger than the adhesive matrix layer and/or may be larger than the backing layer. In yet other embodiments, the release liner may range from about 0.1mm to at least about 20mm larger than the diameter of a round backing layer or a round adhesive matrix layer, preferably ranging from about 0.5mm to about 10mm larger than the backing layer or adhesive matrix layer, and most preferably ranging from about 1mm to about 5mm larger than the backing layer or adhesive matrix layer. The release liner may also range from about 0.1mm to at least about 20mm larger than each side of a rectangular or square backing layer or adhesive matrix layer, preferably ranging from about 0.5mm to about 10mm larger than the backing layer or adhesive matrix layer, and most preferably ranging from about 1mm to about 5mm larger than the backing layer or adhesive matrix layer.

In addition, the release liner may be the same size or larger than an overlay or reservoir liner layer, as discussed herein.

Use of an oversized release liner helps prevent the adhesive matrix from becoming distorted or relaxing during the handling and shipping processes. Such an oversized release liner may help prevent crystal growth, especially when the transdermal delivery devices are stored for long periods of time, are exposed to temperature fluctuations, or are exposed to shipping and/or moving stresses. For example, when an adhesive matrix is laminated between a backing layer and a release liner that is the same size as the adhesive matrix, coupled with heat curing, crystal growth is observed to start from the edge of the patch and progress toward the center. However, when that same adhesive matrix is laminated between a backing layer and an oversized release liner, coupled with heat curing, there is no observed crystal growth even after the patch is stored for two months, subjected to ten cycles of freeze and thaw stability testing, or subjected to repeated microscopic observations.

In some embodiments, no release liner is used and crystal growth is prevented through use of a protective shipping pouch as discussed herein.

In one embodiment, the adhesive matrix layer is laminated between an oversized release liner and an oversized backing layer. In another embodiment, the adhesive matrix layer is laminated between an oversized release liner and backing layer of the same size as the adhesive layer. In yet another embodiment, the adhesive matrix layer is laminated between an oversized release liner and a backing layer of the same size as the adhesive layer with an overlay film above the backing layer. If an overlay film is utilized, the overlay may be the same material or may be a different material than the release liner. Generally, the overlay film is used to prevent adhesive cold flow, *i.e*. is to prevent adhesive material from flowing and contacting the pouch material. It is also believed that the overlay can protect the adhesive edge from contacting the pouch material.

The overlay is typically the same size as the oversized release liner, but larger in size than the backing layer. The overlay layer may be about 0.01mm to at least about 20mm larger than the diameter of a round backing layer or than each dimension of a rectangular or square backing layer. Moreover, the overlay typically covers the edge of the backing layer. Examples of overlay films include polyester films, polyurethanes films, fluoropolymer coated polyester films, fluoropolymer coated polypropylene films, silicone coated polyester films, silicone coated polyurethane films, silicone coated polypropylene films, biaxially oriented polypropylene films, and silicone coated biaxially oriented polypropylene films. Specific examples of overlay films include 3M™ Scotchpak™ 1020, 1022, 9741, 9742, and 9744 (all available from 3M, 3M Corporate Headquarters, 3M Center, St. Paul, MN 55144-1000); CPFilms ClearSIL UV5A (available from CPFilms Inc., PO Box 5068, Martinsville, VA 24115 USA); and Medirelease®2249 and Medirelease® 2226 (available from Mylan Technologies Inc., 110 Lake Street, St. Albans, VT 05478).

The transdermal delivery device may further comprise an underlay layer below the release liner. In general, the underlay layer is used to protect any exposed adhesive on the back of a release liner which has been slit, thereby preventing the adhesive material, containing the active agent, from contacting the pouch material.

In some embodiments, the transdermal delivery device contains both an underlay layer and an overlay layer. In some embodiments, the transdermal delivery device contains an underlay layer without the addition of an overlay layer.

The underlay layer is generally comprised of the same materials as the overlay layer described above. The underlay layer may be the same size as or larger than the release liner.

The transdermal delivery device may include one or more additional layers. One such additional layer is a reservoir layer. Preferably, the reservoir layer, like the other layers described herein, is composed of materials that are free of crystallization seeding particles. Any suitable release liner known in the art may be used. Examples can be found in United States Patent Nos. 6,746,689 and 7,244,447, the disclosures of each are hereby incorporated by reference. The reservoir layer may contain one or more active agents and one or more pharmaceutically acceptable additives.

In general, the reservoir layer is a layer that is placed between a backing film and a drug release regulating membrane layer. In such an example, the reservoir layer contains an amount of active agent which is higher than an amount of active agent present in an adhesive matrix layer (which is located between the membrane layer and the release liner). The active agent(s) may be in amorphous form in an adhesive matrix or in a gel in the reservoir layer. The skin contact layer may include no active agent or may include at least one active agent substantially in amorphous form.

The transdermal delivery system may also include a drug release regulating membrane layer as known in the art, which may be used to control the rate at which an API permeates out of the device. Such a membrane layer may be present in a drug delivery device beneath, and typically immediately adjacent to, the drug reservoir layer, and generally between the drug reservoir itself and an adhesive matrix layer which affixes the device to the skin.

Representative materials useful for forming rate-controlling membrane layers include polyolefins such as polyethylene and polypropylene, polyamides, polyesters, ethylene-ethacrylate copolymers, ethylene-vinyl acetate copolymers, ethylene-vinyl methylacetate copolymers, ethylene-vinyl ethylacetate copolymers, ethylene-vinyl propylacetate copolymers, polyisoprene, polyacrylonitrile, ethylene-propylene copolymers, ethylene-vinyl acetate copolymer, and the like. Other rate-controlling membranes are disclosed in United States Patent No. 7,244,447, the disclosure of which is hereby incorporated by reference. Preferably, the drug release regulating membrane layer is composed of materials that are non-crystallization promoting and free of crystallization nuclei.

The drug release regulating membrane layer may contain one or more active agents and one or more pharmaceutically acceptable additives.

The transdermal delivery device unit dosage form may be placed in appropriate packaging for storage and protection, such as paper, polymer films, and/or metal foil pouches, until they are to be applied in transdermal treatment. The packaging or pouch may be the same size or larger than the overlay or release liner in one or all of the dimensions. The packing or pouch may range from about 0.1mm to about 20mm larger than the overlay and/or release liner, preferably ranging from about 0.2mm to 10mm larger than the overlay and/or release liner, most preferably ranging from about 0.5mm to about 2mm larger than the overlay and/or release liner. A tight fit between the patch and pouch prevents movement of the patch inside the pouch and thus prevents the adhesive edge of the patch from being damaged during shipping and/or handling processes.

Two methods of preparing an adhesive matrix containing at least one active agent that is supersaturated and present in an amorphous form are provided.

A first method comprises the following steps: first, the active agent and an adhesive polymer are dissolved in a solvent system so as to provide the active agent in an adhesive matrix solution at a subsaturated concentration (but once the solvent is removed, the active agent will be at a supersaturated concentration in the dry adhesive matrix); second, the subsaturated active agent in the adhesive matrix solution is cast to at least one of a release liner or a backing layer; third, the solvent is removed from the adhesive matrix solution at a temperature which is at, below, or above the melting point of the active agent to spontaneously form the supersaturated concentration of amorphous drug-in-adhesive matrix; and fourth, the other of a release liner or a backing film is laminated to the supersaturated active agent in the adhesive matrix, so that the supersaturated active agent in the adhesive matrix is between the release liner and the backing layer. Here, the active agent is rotigotine.

In one embodiment of this first method, the release liner and/or the backing layers are non-crystallization promoting and free of crystallization nuclei. In another embodiment of this first method, the supersaturated drug-in-adhesive-matrix contains one or more additives or excipients which are dissolved or undissolved but dispersed as liquid or solid particles in the adhesive matrix. The amount of solvent necessary for this method ranges from about 1% to about 200% more than the amount of solvent necessary to solubilize the drug and adhesive. The solvent may be chosen from organic solvents including pentanes, hexanes, heptanes, octanes, ethyl acetate, ethanol, isopropanol, toluene, xylenes and mixtures thereof. For the adhesive system, if the type of solvent present in the adhesive matrix solution has a lower solubility for the drug than for the adhesive, a second solvent may be added to dissolve both the drug and the adhesive. The ratio of the first solvent to the second solvent is the ratio at which both the adhesive and the drug can be completely dissolved to form a single phase. An optimum ratio and the amount of each of the two solvents required to form a single phase solution of the adhesive and drug varies from drug to drug and varies with the amount of the drug utilized.

A second method of preparing an adhesive matrix containing at least one active agent that is supersaturated and present in amorphous form comprises the following steps: admixing the active agent with an adhesive matrix at a supersaturated concentration; heating the adhesive matrix to a temperature which allows the active agent to be completely dissolved in the adhesive melt, or melted and finely dispersed in the adhesive matrix, to create a hot melt; casting the hot melt to at least one of a release liner or a backing layer; and laminating the other of a release liner or a backing layer to the hot melt, so that the hot melt is between the release liner and the backing layer. As the hot melt is cooled to ambient temperature, the amorphous drug-in-adhesive matrix is spontaneously formed, whereby the solid amorphous drug is finely dispersed in the adhesive matrix. Here, the active agent is rotigotine.

In one embodiment of this method, the hot melt contains one or more additives or excipients which are dissolved or undissolved but dispersed in the adhesive matrix.

In another embodiment of this method, the release liner and the backing layer are non-crystallization promoting and free of crystallization nuclei.

Crystalline forms of drugs are the most thermodynamically stable forms. As a result, drug molecules will self-organize themselves in such a structurally ordered way as to form crystals with the lowest possible amount of energy. Under thermodynamically favored conditions, amorphous forms of drugs or less favored crystal forms will eventually convert to the most stable crystal form. One way in which crystallization or conversion may occur is through the presence of pre-existing drug crystals or other solid particles (nuclei) present in the adhesive matrix which provide support for crystal growth formation. This process is termed crystal seeding. Thus, to avoid crystal growth formation, a backing layer and/or a release liner that are non-crystallization promoting and free of crystallization nuclei is utilized. Such a non-crystallization promoting backing layer and/or a non-crystallization release liner has been shown to prevent crystal formation and growth in an amorphous drug-in-adhesive-matrix. Moreover, utilization of an oversized backing layer or an oversized release liner in a patch may further avoid crystallization of the amorphous form. Indeed, use of such an oversized release liner or oversized backing layer helps prevent the edge of the adhesive matrix from becoming distorted or relaxing during the handling and shipping processes or when the devices are stored for long periods of time or are exposed to temperature fluctuations.

A solid drug can exist in one or more crystalline forms and in amorphous form. Structurally ordered molecules form crystals. Of all the possible crystalline forms, one crystalline form is most thermodynamically stable among the crystalline forms. The amorphous form of a drug, however, is meta stable, meaning it is thermodynamically unstable. Unlike crystalline forms, amorphous drug molecules are structurally organized in a random order. Under thermodynamically favored conditions, the less stable crystalline forms and amorphous form will eventually convert to the most stable crystalline form. Precisely how long a drug retains the meta stable amorphous form before crystallization initiates is dependent on the internal and external environments. Favored external environment conditions include storing an amorphous drug product at a low temperature, e.g., storing the amorphous form of the drug at a temperature that is not more than 50°C higher than its T_{g}, and not disturbing the matrix containing the amorphous drug. Favored internal environments that can extend the life of the amorphous form include those adhesive matrix types that can reduce the movement of amorphous drug molecules by forming hydrophobic associations and/or hydrogen bonds between the matrix molecules and drug molecules.

It is desirable to be able to melt or redissolve the crystallization nuclei and reestablish the internal adhesive matrix for a drug in amorphous form should crystallization initiate. As such, a method of reestablishing the internal adhesive matrix environment for the amorphous form comprises heat curing a die-cut patch at a particular temperature for a sufficient period of time. Preferably, the heat curing is done at the temperature of the melting point of the active agent up to a temperature about 20°C above the melting point of the active agent. Preferably, the heat curing is done either after die-cutting and after packaging or after die-cutting and before packaging. Preferably, heat curing is performed before any crystals are formed or before a substantial amount of crystals are formed. Heat curing sources include oven electric heating and infra-red beams.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

Aspects of the present invention are also defined by way of the following clauses:
1. A transdermal delivery device comprising:
   a) a backing layer,
   b) a release liner, and
   c) an adhesive matrix layer between said backing layer and said release liner comprising a supersaturated concentration of rotigotine substantially in amorphous form within said adhesive matrix,
   wherein one of said release liner or backing layer is larger than said adhesive matrix layer.
2. The transdermal delivery device of clause 1, wherein said rotigotine is present in an amount of about 0.1% to about 50% by weight of said adhesive matrix.
3. The transdermal delivery device of clause 1, wherein the concentration of said rotigotine is from about 0.1% to about 10000% above the solubility of said active agent in said adhesive matrix.
4. The transdermal delivery device of clause 1, wherein said backing layer and said release liner are substantially non-crystallization inducing and free of crystallization nuclei or crystallization seeding particles.
5. The transdermal delivery device of clause 4, wherein said backing layer is selected from the group consisting of polyester films, polyethylene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films.
6. The transdermal delivery device of clause 5, wherein said backing layer is polyester.
7. The transdermal delivery device of clause 1, wherein said backing layer is at least about 0.01mm larger than said adhesive matrix layer.
8. The transdermal delivery device of clause 7, wherein said backing layer is about 0.01mm to about 10mm larger than said adhesive matrix layer.
9. The transdermal delivery device of clause 8, wherein said backing layer is about 0.05mm to about 5mm larger than said adhesive matrix layer.
10. The transdermal delivery device of clause 9, wherein said backing layer is about 0.1mm to about 3mm larger than said adhesive matrix layer.
11. The transdermal delivery device of clause 1, wherein said adhesive matrix layer comprises an adhesive material selected from the group consisting of polyisobutylene, polysiloxane, acrylic adhesives, natural and synthetic rubber adhesives, and mixtures thereof.
12. The transdermal delivery device of clause 11, wherein said adhesive material is present in an amount of from about 50% to about 99% by weight of said adhesive matrix layer.
13. The transdermal delivery device of clause 1, wherein said adhesive matrix layer further comprises one or more tackifiers.
14. The transdermal delivery device of clause1, wherein said adhesive matrix layer further comprises one or more cohesive enhancers.
15. The transdermal delivery device of clause 1, wherein said adhesive matrix layer further comprises one or more flux enhancers.
16. The transdermal delivery device of clause 1, further comprising a drug release regulating membrane layer and a reservoir layer.
17. The transdermal delivery device of clause 16, wherein at least one of said drug release regulating membrane layer and said reservoir layer contains said rotigotine.
18. The transdermal delivery device of clause 1, further comprising an overlay film in communication with said backing layer.
19. The transdermal delivery device of clause 18, wherein said overlay film is larger than said backing layer.
20. The transdermal delivery device of clause 19, wherein said overlay film is about 0.01mm to about 20mm larger than said backing layer.
21. The transdermal delivery device of clause 11, wherein said release liner is larger than said adhesive matrix layer.
22. A transdermal delivery device comprising:
   a) an overlay film,
   b) a backing layer adjacent to said overlay film,
   c) an adhesive matrix layer adjacent to said backing layer comprising a supersaturated concentration of rotigotine substantially in amorphous form within said adhesive matrix, and
   d) a release liner adjacent to said adhesive matrix layer.
23. The transdermal delivery device of clause 22, wherein said overlay film is larger than said backing layer.
24. The transdermal delivery device of clause 23, wherein said overlay film is at least 0.01mm larger than said backing layer.
25. The transdermal delivery device of clause 24, wherein said overlay film is between about 0.01mm to about 20mm larger than said backing layer.
26. The transdermal delivery device of clause 22, wherein said overlay film covers at least one edge of said backing layer.
27. The transdermal delivery device of clause 22, wherein said overlay film is selected from the group consisting polyester films, polyurethane films, polyester films with a silicone coating, polyurethane films with a silicone coating, polyester films with a fluorosilicone coating, polyurethane films with a fluorosilicone coating, silicon coated polyester films, silicon coated polyurethane films, polyester films with a fluoropolymer coating, and polyurethane films with a fluoropolymer coating.
28. The transdermal delivery device of clause 22, wherein said backing layer and said release liner are substantially non-crystallization inducing and free of crystallization nuclei or crystallization seeding particles.
29. The transdermal delivery device of clause 28, wherein said backing layer is selected from the group consisting of polyester films, polyethylene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films.
30. The transdermal delivery device of clause 22, wherein said rotigotine is present in an amount of about 0.1% to about 50% by weight of said adhesive matrix.
31. The transdermal delivery device of clause 28, wherein at least one of said backing layer or release liner is larger than said adhesive matrix layer.
32. The transdermal delivery device of clause 31, wherein said backing layer is the same size as said adhesive matrix layer.
33. The transdermal delivery device of clause 31, wherein said backing layer is larger than said adhesive matrix layer.
34. The transdermal delivery device of clause 31, wherein said release liner is larger than said adhesive matrix layer.
35. The transdermal delivery device of clause 22, wherein said adhesive matrix layer comprises an adhesive material selected from the group consisting of polyisobutylene, polysiloxane, acrylic adhesives, natural and synthetic rubber adhesives, and mixtures thereof.
36. The transdermal delivery device of clause 35, wherein said adhesive material is present in an amount of from about 50% to about 99% by weight of said adhesive matrix layer.
37. The transdermal delivery device of clause 22, further comprising a drug release regulating membrane layer and a reservoir layer.
38. A transdermal delivery device comprising:
   a) a polyester backing layer,
   b) a substantially non-crystallization inducing release liner, and
   c) an adhesive matrix layer between said backing layer and said release liner comprising a supersaturated concentration of rotigotine substantially in amorphous form within said adhesive matrix.
39. The transdermal delivery device of clause 38, wherein at least one of said polyester backing layer or said release liner is larger than said adhesive matrix layer.
40. The transdermal delivery device of clause 38, further comprising an overlay film adjacent to said polyester backing layer and opposite said adhesive matrix layer.
41. The transdermal delivery device of clause 40, wherein said overlay film is larger than said polyester backing layer.
42. A transdermal delivery device comprising:
   a) an overlay film,
   b) a substantially non-crystallization inducing backing layer adjacent to said overlay film,
   c) an adhesive matrix layer adjacent to said backing layer comprising a supersaturated concentration of rotigotine substantially in amorphous form within said adhesive matrix, and
   d) a substantially non-crystallization inducing release liner adjacent to said adhesive matrix layer,
   wherein at least one of said overlay film, backing layer, or release liner is larger than said adhesive matrix layer in at least one dimension.
43. The transdermal delivery device of clause 42, wherein said backing layer is larger than said adhesive matrix layer.
44. The transdermal delivery device of clause 42, wherein said overlay film is larger than said adhesive matrix layer.
45. The transdermal delivery device of clause 42, wherein said release liner is larger than said adhesive matrix layer.
46. The transdermal delivery device of clause 42, wherein said overlay film is made from polyester films, polyurethanes films, fluoropolymer coated polyester films, fluoropolymer coated polypropylene films, silicone coated polyester films, silicone coated polyurethane films, silicone coated polypropylene films, biaxially oriented polypropylene films, and silicone coated biaxially oriented polypropylene films.
47. The transdermal delivery device of clause 42, wherein said backing layer is selected from the group consisting of polyester films, polyethylene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films.
48. A transdermal delivery device comprising:
   a) a substantially non-crystallization inducing backing layer, and
   b) an adhesive matrix layer adjacent to said backing layer comprising a supersaturated concentration of rotigotine substantially in amorphous form within said adhesive matrix, wherein said backing layer is larger than said adhesive matrix layer.
49. The transdermal delivery device of clause 48, further comprising a substantially non-crystallization inducing release liner adjacent to said adhesive matrix layer.
50. The transdermal delivery device of clause 48, wherein said device is stored, transported or protected in protective packaging prior to use.
51. The transdermal delivery device of clause 50, wherein said protective packaging is a pouch comprised of paper, polymer film, metal foil or combinations thereof.
52. The transdermal delivery device of clause 48, further comprising an overlay film adjacent to said backing layer and opposite said adhesive matrix layer.
53. The transdermal delivery device of clause 52, wherein said overlay film is larger than at least one of said backing layer or said adhesive matrix layer.
54. A transdermal delivery device comprising:
   a) a substantially non-crystallization inducing backing layer,
   b) a substantially non-crystallization inducing release liner, and
   c) an adhesive matrix layer between said backing layer and said release liner comprising a supersaturated concentration of rotigotine substantially in amorphous form;
   wherein at least one of said backing layer or release liner is larger than said adhesive matrix layer, and
   wherein said transdermal delivery device is cured at a temperature above the melting point of said rotigotine.
55. The transdermal delivery device of clause 54, wherein said curing is performed at a temperature about 20°C above the melting point of said rotigotine.
56. The transdermal delivery device of clause 54, wherein said curing is performed for a duration ranging from about 1 second to about 10 minutes.
57. The transdermal delivery device of clause 56, wherein said duration ranges from about 3 seconds to about 5 minutes.
58. The transdermal delivery device of clause 54, further comprising an overlay film adjacent to said backing layer and opposite said adhesive matrix layer.
59. The transdermal delivery device of clause 58, wherein said overlay film is larger than said adhesive matrix layer.
60. A transdermal delivery device comprising:
   a) a backing layer,
   b) a release liner, and
   c) an adhesive matrix layer between said backing layer and said release liner comprising a supersaturated concentration of rotigotine substantially in amorphous form within said adhesive matrix,
   wherein said backing layer is the same size as said adhesive matrix layer.
61. The transdermal delivery device as in any one of clauses 1, 22, 38, 42, 54, and 60, further comprising an underlay layer below the release liner.

## Claims

1. A transdermal delivery device comprising:
a) a backing layer,
b) a release liner, and
c) an adhesive matrix layer between said backing layer and said release liner comprising a supersaturated concentration of rotigotine substantially in amorphous form within said adhesive matrix,
wherein one of said release liner or backing layer is larger than said adhesive matrix layer.

2. The transdermal delivery device of claim 1, wherein said rotigotine is present in an amount of about 0.1% to about 50% by weight of said adhesive matrix.

3. The transdermal delivery device of claim 1, wherein the concentration of said rotigotine is from about 0.1% to about 10000% above the solubility of said active agent in said adhesive matrix.

4. The transdermal delivery device of claim 1, wherein said backing layer and said release liner are substantially non-crystallization inducing and free of crystallization nuclei or crystallization seeding particles.

5. The transdermal delivery device of claim 4, wherein said backing layer is selected from the group consisting of polyester films, polyethylene films, metal films, metalized polyester films, nylon films, ethylene vinyl acetate films laminated to a polyester, ethylene vinyl acetate films laminated to a metalized polyester, polyvinylidene fluoride films, silicone coated polyester films, silicone coated polyolefin films, and silicone coated ethyl vinyl acetate films.

6. The transdermal delivery device of claim 5, wherein said backing layer is polyester.

7. The transdermal delivery device of claim 1, wherein said backing layer is at least about 0.01mm larger than said adhesive matrix layer.

8. The transdermal delivery device of claim 7, wherein said backing layer is about 0.01mm to about 10mm larger than said adhesive matrix layer.

9. The transdermal delivery device of claim 8, wherein said backing layer is about 0.05mm to about 5mm larger than said adhesive matrix layer.

10. The transdermal delivery device of claim 9, wherein said backing layer is about 0.1mm to about 3mm larger than said adhesive matrix layer.

11. The transdermal delivery device of claim 1, wherein said adhesive matrix layer comprises an adhesive material selected from the group consisting of polyisobutylene, polysiloxane, acrylic adhesives, natural and synthetic rubber adhesives, and mixtures thereof.

12. The transdermal delivery device of claim 11, wherein said adhesive material is present in an amount of from about 50% to about 99% by weight of said adhesive matrix layer.

13. The transdermal delivery device of claim 1, wherein said adhesive matrix layer further comprises one or more tackifiers.

14. The transdermal delivery device of claim 1, wherein said adhesive matrix layer further comprises one or more cohesive enhancers.

15. The transdermal delivery device of claim 1, wherein said adhesive matrix layer further comprises one or more flux enhancers.
